# EUROPEAN PATENT APPLICATION

(11) **EP 3 561 550 A1**
(43) Date of publication of application: **30.10.2019**
(21) Application number: 17885631.6
(22) Date of filing: 07.11.2017
(51) Int. Cl.: G01V 5/00, G01N 23/10

(54) **MOBILE BOMB DISPOSAL TRANSMISSION IMAGING APPARATUS**

(30) Priority: 26.12.2016 CN 201611220520
(71) Applicant: Nuctech Company Limited, Beijing 100084 (CN)
(72) Inventor: LIN, Dong, Beijing 100084 (CN); CUI, Jin, Beijing 100084 (CN); TAN, Xianshun, Beijing 100084 (CN); ZHANG, Qing, Beijing 100084 (CN)
(74) Representative: Cabinet Beau de Loménie
(86) International application number: PCT/CN2017/109692
(87) International publication number: WO 2018/121081

(57) **Abstract**

A mobile bomb disposal transmission imaging apparatus (100), comprising a mobile base plate (110), a detection arm mechanism (120), a ray apparatus (130), and a receiving imaging apparatus. The mobile base plate (110) has a travelling mechanism; the detection arm mechanism (120) is arranged on the mobile base plate (110) and is capable of switching between an expanded state and a folded state, the detection arm mechanism (120), when in an expanded state, forming a gate structure that spans the top and two sides of the object (200) to be detected; the ray apparatus (130) is arranged on the mobile base plate (10), a ray beam emitted thereby forming a ray beam surface for transmitting through the object (200) to be detected; the receiving imaging apparatus is arranged on the detection arm mechanism (120) and, when the detection arm mechanism (120) is in an expanded state, receives the ray beam transmitting through the object (200) to be detected emitted by the ray apparatus (130), and creates a detection image.

## Description

### CROSS REFERENCE

This application claims priority to Chinese Patent Application No. 201611220520.9, filed on December 26, 2016, the entire contents of which are incorporated herein by reference.

### TECHNICAL FIELD

The present disclosure relates to a radiation imaging technical field, in particular to a mobile explosive-removal transmission imaging apparatus.

### BACKGROUND

With development of science and technology in public security, the existing security inspection devices are widely used in civil aviation, railway, subway, highway and other fields for security inspection. For example, for inspection to luggage packages, the security inspection device usually used includes a fixed baggage X-ray security inspection instrument, a fixed industrial CT, a Raman spectral imager, a trace detector, etc. The aforesaid security inspection devices can carry out security checks, such as anti-terrorism, anti-riot and anti-virus, on most of the luggage packages. However, to use the security inspection devices, it is required to put the luggage packages into the corresponding position of the security inspection device, or to handheld the security inspection instrument close to items to be checked. When finding a suspected object separately placed on the ground to be checked, especially the luggage packages suspected of explosion, a security guard can hardly approach to the luggage package for inspection. As above described, it is an urgent need in this field to provide a mobile explosive-removal inspection device instead of the security guard to approach to the suspected luggage packages for inspection.

### SUMMARY

An important object of the present disclosure is to overcome at least one disadvantage in the prior art. It is provided with a mobile explosive-removal transmission imaging apparatus with an automatic detection and mobile function.

To realize the above object, the present disclosure adopts following technical solution: According to one aspect of the present disclosure, a mobile explosive-removal transmission imaging apparatus for transmission imaging on an object to be detected, wherein the mobile explosive-removal transmission imaging apparatus includes a mobile chassis, a detection arm mechanism, a radiation device, and a receiving imaging device. The mobile chassis includes a walking mechanism. The detection arm mechanism is arranged on the mobile chassis and capable of being switched between an unfolded state and a folded state. The detection arm mechanism as being in the unfolded state is a door-shaped structure spanning over a top and two sides of the object to be detected. The radiation device is provided on the mobile chassis, and ray beam emitted by the radiation device forms a ray beam surface that transmits the object to be detected. The receiving imaging device is arranged on the detection arm mechanism to receive the ray beam emitted by the radiation device and transmitting through the object to be detected, and produce a detection image, when the detection arm mechanism is in the unfolded state.

According to one of implementations of the present disclosure, the detection arm mechanism includes a post, a transverse detection arm, a vertical detection arm and a folding mechanism. The post is vertically and rotatably arranged on the mobile chassis.

The transverse detection arm has one end fixed to a top end of the post and the other end extending horizontally. The vertical detection arm has one end connected to the other end of the transverse detection arm. The folding mechanism is disposed between the transverse detection arm and the vertical detection arm to open the vertical detection arm to form the door-shaped structure, or fold the vertical detection arm. Wherein, the receiving imaging device are respectively arranged on the transverse detection arm and the vertical detection arm.

According to one of implementations of the present disclosure, the post includes a fixing part, a lifting part and a lifting drive device. The fixing part is rotatably arranged on the mobile chassis. The lifting part is movably up and down arranged on the fixing part. One end of the transverse detection arm is fixed to the top end of the lifting part. The lifting drive device is used for driving the lifting part to lift up and down on the fixing part.

According to one of implementations of the present disclosure, the lifting drive device includes a first thrusting part. The first operating part is vertically arranged and has a first guide sleeve and a first thrusting rod. The first guide sleeve and the first thrusting rod are respectively connected to the fixing part and the lifting part of the post.

According to one of implementations of the present disclosure, the post includes at least two lifting drive devices that are respectively arranged on opposite sides of the fixing part.

According to one of implementations of the present disclosure, the folding mechanism includes a hinge and a folding drive device. The hinge is rotatably connected between the other end of the transverse detection arm and one end of the vertical detection arm. The folding drive device is used for driving the vertical detection arm to be switched between a vertical state and a horizontal state.

According to one of implementations of the present disclosure, the folding drive device includes a second thrusting part. The second thrusting part has a second guide sleeve and a second thrusting rod. The second guide sleeve and the second thrusting rod are respectively connected to the transverse detection arm and the vertical detection arm), or, the second guide sleeve and the second thrusting rod are respectively connected to the vertical detection arm and the transverse detection arm.

According to one of implementations of the present disclosure, the detection arm mechanism further includes a turntable that is arranged on the mobile chassis, wherein the post is arranged on the turntable.

According to one of implementations of the present disclosure, the mobile chassis is provided with a supporting mechanism to carry the detection arm mechanism when the detection arm mechanism is in a folded state.

According to one of implementations of the present disclosure, the mobile chassis includes a main body and a bracket. The main body is provided with the walking mechanism. The bracket is arranged on one side of the main body. The detection arm mechanism is arranged on the bracket.

According to one of implementations of the present disclosure, the radiation device includes a rotating frame and a ray generator. The rotating frame is rotatably provided on the mobile chassis. The ray generator is arranged on the rotating frame. Wherein when the mobile explosive-removal transmission imaging apparatus performs a transmission imaging on the object to be detected, the rotating frame rotates relative to the mobile chassis to change a height of the ray generator to adjust a coverage of the ray beam surface.

According to one of implementations of the present disclosure, the walking mechanism is a track.

As can be seen from the aforesaid technical solution, advantages and advantageous effects of the mobile explosive-removal transmission imaging apparatus proposed in the present disclosure are in that:
The present disclosure provides a mobile explosive-removable transmission imaging apparatus, which may complete detection on the suspected items through the remote control, and thus meeting the requirements for security inspection to the suspected luggage packages that cannot allow the security guard to be approached. The present disclosure adopts a non-contact transmission imaging technology without need to direct contact with the object to be detected, and thus reducing probability of dangerous situations happened during the detection. In addition, the detection arm mechanism of the present disclosure is a foldable structure, so that in the non-work state, it can reduce its volume, increase stability and facilitate for transportation and self-movement within a short distance

### BRIEF DESCRIPTION OF THE DRAWINGS

The various objects, features and advantages of the present disclosure will be apparent from the following detailed description of the preferable embodiments taken in conjunction with the accompanying drawings. The figures of the present disclosure are only illustrative, but not necessarily to scale. In the drawings, the same reference numbers will be used throughout the drawings to refer to the same or like parts, in which,
FIG. 1 is a schematic structural perspective view of a mobile explosive-removal transmission imaging apparatus in a working state according to an exemplary implementation.
FIG. 2 is a partial enlargement view of the mobile explosive-removal transmission imaging apparatus as shown in FIG. 1;
FIG. 3 is a schematic structural perspective view of the mobile explosive-removal transmission imaging apparatus in a non-working state as shown in FIG. 1.

Wherein, the reference numbers are listed as follows:
- 100: mobile explosive-removal transmission imaging apparatus;
- 110: mobile chassis;
- 111: main body;
- 112: bracket;
- 113: track;
- 114: supporting mechanism;
- 120: detection arm mechanism;
- 121: post;
- 1211: fixing part;
- 1212: lifting part;
- 1213: first thrusting part;
- 122: transverse detection arm;
- 123: vertical detection arm;
- 124: folding mechanism;
- 1241: hinge;
- 1242: second thrusting part;
- 125: turntable;
- 130: radiation device;
- 131: rotating frame;
- 132: ray generator;
- S: ray beam surface;
- 200: object to be detected

### DETAILED DESCRIPTION

Typical embodiments embodying features and advantages of this disclosure will be set forth below in detail. It should be understood that various modifications may be made in different embodiments without departing from the scope of this disclosure, wherein the specification and drawings in essential are used for description but not limit to this disclosure.

Hereinafter, various exemplary implementations of the present disclosure will be described with reference to the drawings that constitute one part of the present disclosure, in which different exemplary structures, systems and steps of various aspects of the present disclosure can be realized in an exemplary example. It should be understood that other specific technical solutions of the components, structures, exemplary devices, systems, and steps may be used and can be structurally and functionally modified without departing from the scope of this disclosure. Moreover, although the terms "top end", "bottom end", "between", "side" etc. may be used in this specification to describe different exemplary features and elements of the present disclosure, these terms are used herein only for convenience, for example, the exemplary direction as described according to the drawings. It should not be understood from any content of the specification that particular three-dimensional direction requiring a structure falls within the scope of the present disclosure.

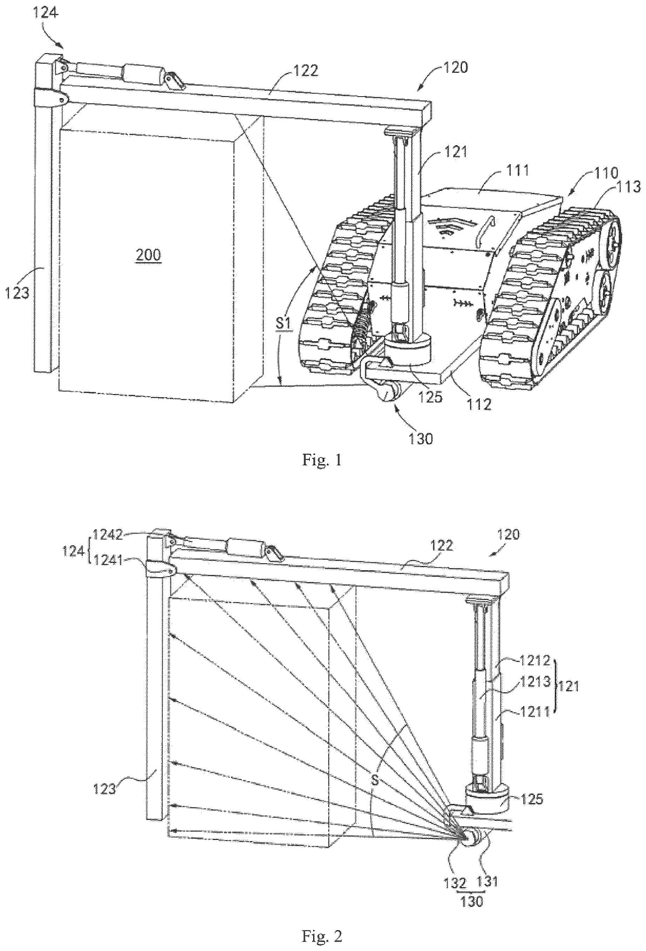

Referring to FIG. 1, a mobile explosive-removal transmission imaging apparatus 100 capable of embodying principles of the present disclosure is representatively shown. In this exemplary implementation, the mobile explosive-removal transmission imaging apparatus 100 as provided in this disclosure is illustrated by taking a detection device for transmission imaging detection on an object to be inspected as an example. It is easy for those skilled in the art to understand that various modifications, additions, substitutions, deletions or other changes are made to the following specific embodiments, which are still within the principles of the mobile explosive-removal transmission imaging apparatus 100 provided in this disclosure, in order to apply the mobile explosive-removal transmission imaging apparatus 100 to other detection fields.

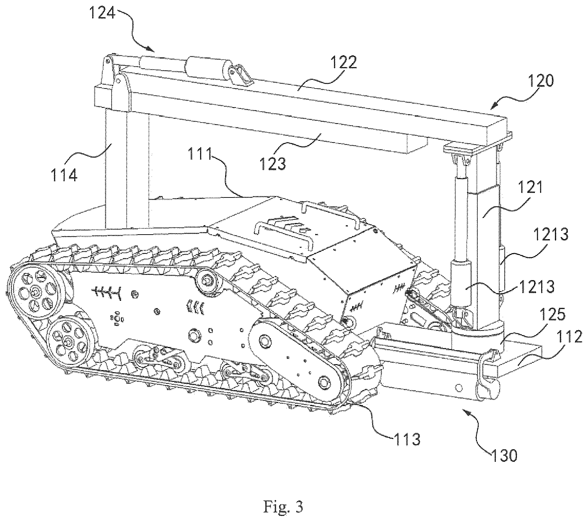

As shown in FIG. 1, a schematic structural perspective view of the mobile explosive-removal transmission imaging apparatus 100 in a working state provided in the present disclosure is specifically shown. In this implementation, the mobile explosive-removal transmission imaging apparatus 100 mainly includes a mobile chassis 110, a detection arm mechanism 120, a radiation device 130, and a receiving imaging device. Referring to FIGS. 2 and 3, FIG. 2 representatively shows a partial enlargement view of the mobile explosive-removal transmission imaging apparatus 100, specifically showing the detection arm mechanism 120 and the radiation device 130. FIG. 3 representatively shows a schematic structural perspective view of the mobile explosive-removal transmission imaging apparatus 100 in a non-working state. In conjunction with the drawings, the main parts of the mobile explosive-removal transmission imaging apparatus 100 provided in the present disclosure will be described in detail.

As shown in FIG. 1, in this implementation, the mobile chassis 110 mainly includes a main body 111 and a bracket 112. Specifically, the main body is provided with tracks 113 on both sides, which can make the mobile explosive-removal transmission imaging apparatus 100 move more stably and can adapt to uneven road surfaces such as steps, ramps and the like to improve trafficability, can expand the range of reachable areas, and does not need a hoisting equipment to hoist it, and thus saving manpower and material resources. In addition, the bracket 112 is arranged at a front end of the main body 111, for example, the bracket 112 may be horizontally extended from a bottom of the front end of the main body 111, to permit the detection arm mechanism 120 and the radiation device 130 to be arranged on the bracket112. It is easy for those skilled in the art to understand that various modifications, additions, substitutions, deletions or other changes are made to a structure and connection relationship of the above-mentioned mobile chassis 110, in order to provide the mobile chassis 110 capable of walking and to provide the detection arm mechanism 120 and the radiation device 130 on the mobile chassis 110. These changes are still within the principles of the mobile explosive-removable transmission imaging apparatus 100 provided in this disclosure. For example, in other exemplary implementations of the present disclosure, the bracket 112 may also be arranged at other positions of the main body 111, or in the absence of the bracket 112, the detection arm mechanism 120 and the radiation device 130 may be arranged at other positions of the mobile chassis 110 or directly arranged on the main body 111, as long as requirements for work and space of the detection arm mechanism 120 and the radiation device 130 are satisfied. Further, in other exemplary implementations of the present disclosure, the track 113 may be replaced by other elements, such as rollers, etc., which herein is not limited thereto.

As shown in FIGS. 1 to 3, in this implementation, the detection arm mechanism 120 is arranged on the bracket112 of the mobile chassis 110, and can be switched between an unfolded state shown in FIG. 1 and a folded state shown in FIG. 3. The detection arm mechanism 120 as being in the unfolded state is presented as a door-shaped structure spanning over a top and two sides of the object to be detected. Specifically, in this implementation, the detection arm mechanism 120 mainly includes a post 121, a transverse detection arm 122, a vertical detection arm 123, a folding mechanism 124, and a turntable 125. The post 121 is vertically and rotatably disposed on the mobile chassis 110. The transverse detection arm 122 has one end fixed to the top of the post 121 and the other end extending horizontally, and one end of the vertical detection arm 123 is connected to the other end of the transverse detection arm 122. The folding mechanism 124 is arranged between the transverse detection arm 122 and the vertical detection arm 123 to open the vertical detection arm 123 to form a door-shaped structure, or fold the vertical detection arm 123. A receiving imaging device are respectively arranged on the transverse detection arm 122 and the vertical detection arm 123 to receive rays emitted by the radiation device 130 and transmitted through the object to be detected, and to display a transmission image of the detected object when the detection arm mechanism 120 is in the unfolded state. The turntable 125 is arranged on the bracket 112 of the mobile chassis 110, and the post 121 is arranged on the turntable 125, so that the post 121 is driven by rotation of the turntable 125, that is, cause rotation of the whole detection arm mechanism 120.

Furthermore, as shown in FIG. 2, in this implementation, post 121 mainly includes a fixing part 1211, a lifting part 1212 and a lifting drive device. Specifically, the fixing part 1211 is rotatably arranged on the mobile chassis 110. The lifting part 1212 is movably up-and-down on the fixing part 1211, and one end of the transverse detection arm 122 is fixed on a top end of the lifting part 1212. The lifting drive device preferably includes a first thrusting part 1213 (e.g., a linear actuator such as a first electric thrusting rod). The first thrusting part 1213 is vertically provided and has a first guide sleeve 12130 and a first thrusting rod 12131. The first guide sleeve 12130 and the first thrusting rod 12131 are respectively connected to the fixing part 1211 and the lifting part 1212 of the post 121 to drive the lifting part 1212 to move up and down on the fixing part 1211. It is easy to understand that the first guide sleeve 12130 and the first thrusting rod 12131 may also be respectively connected to the lifting part 1212 and the fixing part 1211 of the post 121, and may be flexibly selected from the other drive apparatus such as air cylinders and hydraulic cylinders to replace the first thrusting part 1213, which is not limited thereto.

Furthermore, as shown in FIG. 2, in this implementation, the folding mechanism 124 includes a hinge 1241 and a folding drive device. The hinge 1241 is rotatably connected between the other end of transverse detection arm 122 and one end of vertical detection arm 123. The folding drive device preferably includes a second thrusting part 1242 (e.g., a linear actuator such as a second electric thrusting rod). The second thrusting part 1242 has a second guide sleeve 12420 and a second thrusting rod 12421 that are respectively connected to the transverse detection arm 122 and the vertical detection arm 123, to drive the vertical detection arm 123 to be switched between a vertical state and a transverse state. It is easy to understand that the second guide sleeve 12420 and the second thrusting rod 12421 may also be connected to the vertical detection arm 123 and the transverse detection arm 122, respectively, and may be flexibly selected from other drive apparatus such as air cylinders and hydraulic cylinders to replace the second thrusting part 1242, which is not limited thereto.

Those skilled in the art would readily understand that various modifications, additions, substitutions, deletions or other changes are made to the structure, the connection relationship and the selection of the detection arm mechanism 120 in order to permit the detection arm mechanism 120 to form the door-shaped structure spanning over the top and both sides of the detected object and have a switching function between an unfolded state and a folded state. These changes are still within the principles of the mobile explosive-removable transmission imaging apparatus 100 provided by the present disclosure. For example, in other exemplary implementations of the present disclosure, a connection relationship between the end of the transverse detection arm 122 and the top of the post is not limited to relatively fixing, and may also be selected to be hinged or the like, so that the transverse detection arm 122 and the vertical detection arm 123 that are folded together are folded again with respect to the post. As another example, in other exemplary implementations of the present disclosure, the post is not limited to a lifting structure. When the size of the detected object is constant, the post may also be designed as a structure without a lifting function, to further simplify the structure of the apparatus while the functional requirements are satisfied.

As shown in FIGS. 1 to 3, in this implementation, the radiation device 130 is provided on the mobile chassis 110 and mainly includes a rotating frame 131 and a ray generator 132. Specifically, the rotating frame 131 is rotatably disposed on the bracket 112 of the mobile chassis 110, and the ray generator 132 is disposed on the rotating frame 131. Wherein, the rotating frame 131 may preferably be C-shaped or 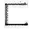 -shaped. The rotating frame 131 has one end portion hinged to an upper surface of the bracket 112, and the other end portion wound from its one side to the lower surface of the bracket 112. Ray generator 132 may be installed on the other end of the rotating frame 13 1. Based on the above configuration, the ray generator 132 is under, beside or over the bracket 112 by the rotation of the rotating frame 131 so that an effect of reducing a detection blind area can be achieved by reducing a height of the ray generator 132 when the mobile explosive-removal transmission imaging apparatus 100 is in operation. When the ray generator132 is in its working position, the ray beam emitted by the ray generator 132 forms a ray beam surface S that is parallel to a length direction of the detection arm mechanism 120 (i.e., a direction in which the transverse detection arm 122 extends horizontally in FIG. 1). The ray beam can transmit the object to be detected and transmit to the vertical detection arm 123 and a part of the transverse detection arm 122. It is easy for those skilled in the art to understand that various modifications, additions, substitutions, deletions, or other changes are made to the structure, connection relationship, and type selection of the above-mentioned radiation device 130 in order to provide the radiation device 130 for transmitting the detected object, and these changes are still within the principles of the mobile explosive-removal transmission imaging apparatus 100 provided in this disclosure. For example, in other exemplary implementations of the present disclosure, the radiation device 130 may also select other structures, for example, by substituting the other structures for the rotating frame 131, or in the absence of the rotating frame 131, when the mobile explosive-removal transmission imaging apparatus 100 performs transmission imaging on the detected object, it is preferable to change the height of the ray generator 132 in other manners to adjust coverage of the ray beam surface S.

In the present implementation, the receiving imaging device is arranged on the detection arm mechanism 120 to receive the ray beam that is emitted by the radiation device 130 and transmits through the object to be detected, and then produce a detection image, when the detection arm mechanism 120 is in an unfolded state. Specifically, on the basis of the structure of the radiation device 130 and a general status of the ray beam surface S in this implementation, the receiving imaging device may be arranged on the vertical detection arm 123 and a part of the transverse detection arm 122 to ensure an imaging function when the ray beam surface S presents a maximum coverage. The receiving imaging device can realize a function of distinguishing whether dangerous and prohibited articles being hidden in the detected object by receiving transmission signal after the ray beam emitted by the radiation device 130 passes through the detected object, and then being imaged through signal processing

It should be noted here that the mobile explosive-removal transmission imaging apparatus 100 as shown in the drawings and as described in this specification is just one example of plenty of mobile explosive-removal transmission imaging apparatus 100 capable of employing the principle of the present disclosure. It should be clearly understood that the principle of the present disclosure is absolutely not only limited to any detail of the evacuation device as shown in the drawings or as described in the specification or any components of the evaporation device.

For example, as shown in FIG. 3, in this implementation, post 121 includes a first thrusting part 1213 with two first thrusting rods 12131, which are respectively mounted on opposite sides of the fixing part 1211 (i.e., a lifting part 1212). Due to the above design, the lifting part 1212 can be forced more evenly as lifting up and down relative to the fixing part 1211, such that the post 120 lifts more stable and reliable. In other exemplary implementations of the present disclosure, more than two lifting drive devices may be provided around the post 120, to further improve stability and reliability of the lifting operation. Of course, post 120 should be equipped with at least one lifting drive device to meet the need for lifting of the post 120.

Furthermore, as shown in FIG. 3, in the present implementation, the mobile chassis 110 is provided with a supporting mechanism 114 (not shown in FIG. 1). The supporting mechanism 14 may preferably be a supporting column with a bracket fixed on the main body 111, to carry the detection arm mechanism 120 as being in a folded state. That is, in this implementation, the folding mechanism 124 drives the vertical detection arm 123 to fold relative to the transverse detection arm 122, and the turntable 125 drives the folded detection arm mechanism 120 to rotate integrally, until the vertical detection arm 123 that has been folded in a substantially transverse state is located above the supporting mechanism 14 and carried by the supporting mechanism 114.

Furthermore, in this implementation, the mobile explosive-removal transmission imaging apparatus 100 provided in the present disclosure may be connected to a remote control system in a wireless or wired manner to control the walking mechanism, the radiation device 130 and the detection arm mechanism 120 of the present disclosure through the remote control system. At the same time, the remote control system may also include an imaging display interface to receive and display the transmission picture information of the detected object received by the receiving imaging device. In other exemplary implementations of the present disclosure, the remote control system may control various parts of the present disclosure in various manners, such as by independently control or integratedly control, and the imaging display interface may also be provided independently, which is not limited thereto.

As above described, the work principle and the work procedure of the mobile explosive-removal transmission imaging apparatus 100 provided by the present disclosure are roughly described as follows:
When need to perform a detection task, the mobile explosive-removal transmission imaging apparatus 100 is transported to a site, and automatically approaches from a position far away from an object to be detected by means of a walking function of its walking mechanism. And, the post 121 (i.e., the detection arm mechanism 120) is controlled to rotate about 90 degrees on the turntable 125 (the detection arm mechanism 120 when being not in work is in the folded state, and the transverse detection arm 122 is located above the main body 111 of the mobile chassis 110 and faces towards its rear end direction), so that the transverse detection arm 122 (including the vertical detection arm 123 folded together with the transverse detection arm 122) is turned to one side above the mobile chassis 110. The first thrusting rod 12131 of the first thrusting part 1213 is controlled to drive the post to rise such that the transverse detection arm 122 rises to a height greater than a length of the vertical detection arm 123, and the first thrusting rod 12131 of the second thrusting part 1242 is controlled to drive the vertical detection arm 123 to rotate to be in a vertical state relative to the transverse detection arm 122, and thereby completing the detection arm mechanism 120 switching from the folded state to the unfolded state. In addition, the rotating frame 131 of the radiation device 130 is controlled to rotate around a rotating shaft to bring the ray generator 132 to the working position. At this time, through the remote control, the mobile explosive-removal transmission imaging apparatus 100 is moved to the object to be detected, and thus the door-shaped structure of the detection arm mechanism 120 spans over the top and both sides of the object to be detected, in this way, the object to be detected is in the coverage area of ray beam surface S, so that an omnidirectional scanning detection on the object to be detected is completed through movement of the mobile explosive-removal transmission imaging apparatus 100 of the present disclosure. After detection, the detection arm mechanism 120 is switched into the folded state, leaving from the detected object or waiting for recovery. Please refer to the above principles and procedures, which will not be described any more.

As above described, the mobile explosive-removable transmission imaging apparatus 100 provided by the present disclosure may complete the detection on the suspected items through the remote control, which meets the requirements for security inspection to the suspected luggage packages that cannot allow the security guard to be approached. The present disclosure adopts a non-contact transmission imaging technology without need to direct contact with the object to be detected, and thus reducing probability of dangerous situations happened during the detection. The detection arm mechanism 120 of the present disclosure is a foldable structure, so that in the non-work state, it can reduce its volume, increase stability and facilitate for transportation and self-movement within a short distance, and such smaller volume is convenient to move and pass through the obstacles such as steps. When the post is designed to be a lifting structure, the mobile explosive-removable transmission imaging apparatus 100 provided by the present disclosure can suit for the objects to be detected with different sizes. Furthermore, based on the above principles, the transverse detection arm 122 may also be designed as a scalable structure, or may be permit to have a function of changing its size through other designs, so that the present disclosure has a wider application space. Exemplary embodiments of the mobile explosive-removal transmission imaging apparatus as proposed by the present disclosure are described and/or illustrated in detail. However, the embodiments of the present disclosure are not limited to the specific embodiments as described herein. Rather, the constituents and/or steps of each embodiment may be used independently and separately from the other constituents and/or steps as described herein. Each constituent and/or step of one embodiment may also be used in combination with other constituents and/or steps of the other embodiments. As introducing the elements/constituents and the like as described and/or shown in the drawings, the terms "a", "an", "the", "said" and "at least one", when describing element/ constituent/ or the like as described and/or shown herein, are used to express the presence of one or more the element/ constitute/ or the like. The terms "include", "comprise" and "have", as used herein, are intended to be inclusive, and mean there may be additional elements/ constituents/ or the like other than the listed elements/ constituents/ or the like. In addition, the words "first" and "second" or the like, as used in claims, are meant to indication, but not to limit the object to which they modify.

Although the mobile explosive-removal transmission imaging apparatus as proposed by the present disclosure is disclosed according to different particular embodiments, those skilled in the art would recognize that the implementations of present disclosure can be modified within the spirit and scope of the claims. The present disclosure is described merely through aforesaid specific implementations, but the protection scope of the present disclosure is not limited thereto. Any person skilled in the art may easily think of variations or substitutions within the technical scope as disclosed in the present disclosure, which all should be contained within the scope of the disclosure. Therefore, the protection scope of the present disclosure should be determined depending on the protection scope of the claims.

## Claims

1. A mobile explosive-removal transmission imaging apparatus (100) for transmission imaging on an object to be detected (200), **characterized in that** the mobile explosive-removal transmission imaging apparatus (100) comprises:
a mobile chassis (110) with a walking mechanism;
a detection arm mechanism (120) arranged on the mobile chassis (110) and capable of being switched between an unfolded state and a folded state, the detection arm mechanism (120) in the unfolded state being a door-shaped structure spanning over a top and two sides of the object to be detected (200);
a radiation device (130) provided on the mobile chassis (110), and a ray beam emitted by the radiation device forming a ray beam surface (S) that transmits the object to be detected (200); and
a receiving imaging device arranged on the detection arm mechanism (120) to receive the ray beam emitted by the radiation device (130) and transmitting through the object to be detected (200), and produce a detection image, when the detection arm mechanism (120) is in the unfolded state.

2. The mobile explosive-removal transmission imaging apparatus (100) according to claim 1, **characterized in that** the detection arm mechanism (120) comprises:
a post (121) vertically and rotatably arranged on the mobile chassis (110);
a transverse detection arm (122) having one end fixed to a top end of the post (121) and the other end extending horizontally;
a vertical detection arm (123) having one end connected to the other end of the transverse detection arm (122); and
a folding mechanism (124) disposed between the transverse detection arm (122) and the vertical detection arm (123) to open the vertical detection arm (123) to form the door-shaped structure, or fold the vertical detection arm (123);
wherein, the receiving imaging devices are respectively arranged on the transverse detection arm (122) and the vertical detection arm (123).

3. The mobile explosive-removal transmission imaging apparatus (100) according to claim 2, **characterized in that** the post (121) comprises:
a fixing part (1211) rotatably arranged on the mobile chassis (110);
a lifting part (1212) movably up and down arranged on the fixing part (1211), and one end of the transverse detection arm (122) being fixed to a top end of the lifting part (1212); and
a lifting drive device for driving the lifting part (1212) to lift on the fixing part (1211).

4. The mobile explosive-removal transmission imaging apparatus (100) according to claim 3, **characterized in that** the lifting drive device comprises a first thrusting part (1213), The first operating part (1213) is vertically arranged and has a first guide sleeve (12130) and a first thrusting rod (12131), and the first guide sleeve (12130) and the first thrusting rod (12131) are respectively connected to the fixing part (1211) and the lifting part (1212) of the post (121).

5. The mobile explosive-removal transmission imaging apparatus (100) according to claim 3, **characterized in that** the post (121) comprises at least two lifting drive devices that are respectively arranged on opposite sides of the fixing part (1211).

6. The mobile explosive-removal transmission imaging apparatus (100) according to claim 2, **characterized in that** the folding mechanism (124) comprises:
a hinge (1241) rotatably connected between the transverse detection arm (122) and one end of the vertical detection arm (123); and
a folding drive device configured to drive the vertical detection arm (123) to be switched between a vertical state and a horizontal state.

7. The mobile explosive-removal transmission imaging apparatus (100) according to claim 6, **characterized in that** the folding drive device comprises a second thrusting part (1242), the second thrusting part (1242) has a second guide sleeve (12420) and a second thrusting rod (12421), and the second guide sleeve (12420) and the second thrusting rod (12421) are respectively connected to the transverse detection arm (122) and the vertical detection arm (123), or the second guide sleeve (12420) and the second thrusting rod (12421) are respectively connected to the vertical detection arm (123) and the transverse detection arm (122).

8. The mobile explosive-removal transmission imaging apparatus (100) according to claim 2, **characterized in that** the detection arm mechanism (120) further comprises a turntable (125) arranged on the mobile chassis (110), wherein the post (121) is arranged on the turntable (125).

9. The mobile explosive-removal transmission imaging apparatus (100) according to any of claims 1-8, **characterized in that** the mobile chassis (110) comprises:
a main body (111) provided with the walking mechanism; and
a bracket (112) arranged on one side of the main body (111), and the detection arm mechanism (120) being arranged on the bracket (112).

10. The mobile explosive-removal transmission imaging apparatus (100) according to any of claims 1-8, **characterized in that** the mobile chassis (110) is provided with a supporting mechanism (114) to carry the detection arm mechanism (120) when the detection arm mechanism (120) is in a folded state.

11. The mobile explosive-removal transmission imaging apparatus (100) according to any of claims 1-8, **characterized in that** the radiation device (130) comprises:
a rotating frame (131) rotatably provided on the mobile chassis (110); and
a ray generator (132) arranged on the rotating frame (131);
when the mobile explosive-removal transmission imaging apparatus (100) performs a transmission imaging on the object to be detected (200), the rotating frame (131) rotates relative to the mobile chassis (110) to change a height of the ray generator (132) to adjust a coverage of the ray beam surface (S).

12. The mobile explosive-removal transmission imaging apparatus (100) according to any of claims 1-8, **characterized in that** the walking mechanism is a track (113).
